# EUROPEAN PATENT APPLICATION

(11) **EP 4 837 602 A1**
(43) Date of publication of application: **11.11.2026**
(21) Application number: 25874635.3
(22) Date of filing: 26.03.2025
(51) Int. Cl.: A61M 5/30, A61M 5/315

(54) **NEEDLELESS INJECTOR**

(30) Priority: 26.03.2024 KR 20240040908
(71) Applicant: Baz Biomedic Co., Ltd., Seoul 06133 (KR)
(72) Inventor: HAM, Hwi-Chan, Seoul 06133 (KR); YU, Woo-Sang, Seoul 06133 (KR); CHO, Sang-Kyung, Seoul 06133 (KR)
(74) Representative: Stöckeler, Ferdinand
(86) International application number: PCT/KR2025/003847
(87) International publication number: WO 2025/206734

(57) **Abstract**

Proposed is a needleless syringe including a driving unit, a conversion unit, and a nozzle unit, in which the driving unit provides a pulse pressure or a pulse-type driving force for reciprocating a power transmission unit accommodated in the housing, the conversion unit converts the pulse pressure or a reciprocating motion of the power transmission unit into an incremental advancing motion of the piston such that, for each pulse of the pulse pressure or each reciprocating motion of the power transmission unit, the piston that has received kinetic energy from the driving unit incrementally advances while stepwise advancing a predetermined distance toward the nozzle unit, and the nozzle unit has a drug reservoir containing a drug therein, and discharges the drug contained in the drug chamber in the form of a microjet through a nozzle hole in accordance with pressure applied to the drug by the piston.

## Description

### [Technical Field]

The present disclosure relates to a needleless syringe and, more specifically, to a needleless syringe that allows for repeated injection of drug at high speed without using a syringe needle.

### [Background Art]

In general, a syringe is a device for injecting a drug solution into the tissue of a living organism. A syringe is composed of a needle that is inserted into the body, a syringe barrel that holds a drug solution, and a piston that pushes the drug solution into the needle by moving forward and backward inside the barrel. A hole is formed in the needle, so that drug is injected during injection.

However, in the case of patients such as diabetics who need to receive injections several times a day, they must receive injections frequently even under home care rather than at a hospital, thus there is difficulty, especially when administering an injection to oneself during self-care at home rather than at a hospital, because of the pain when a needle penetrates the skin.

Accordingly, in recent years, in order to alleviate fear of syringe needles and to prevent infection caused by a needle, research and development on needleless syringes has been actively conducted.

However, since the existing needleless syringes are configured to inject a predetermined amount of drug into a single site of the skin in one shot, damage to skin tissue, etc. may occur.

Further, due to the inconvenience of reloading after a single injection, it has a limitation in that it cannot be used to evenly inject drug multiple times over a wide skin area in fields such as skin aesthetics.

Accordingly, the inventors of the present disclosure have developed a needleless syringe disclosed in Patent Document 1 in order to solve these problems. According to the needleless syringe disclosed in Patent Document 1, a piston that injects drug under pressure is configured to be able to repeatedly reciprocate forward and backward using a power source such as a solenoid, so that it is possible to evenly inject drug over a wider skin area by repeatedly injecting a small amount of drug at high speed.

(Patent Document 1) Patent Document 1: Korean Patent No. 10-2088830 (Mar. 9, 2020)

### [Disclosure]

### [Technical Problem]

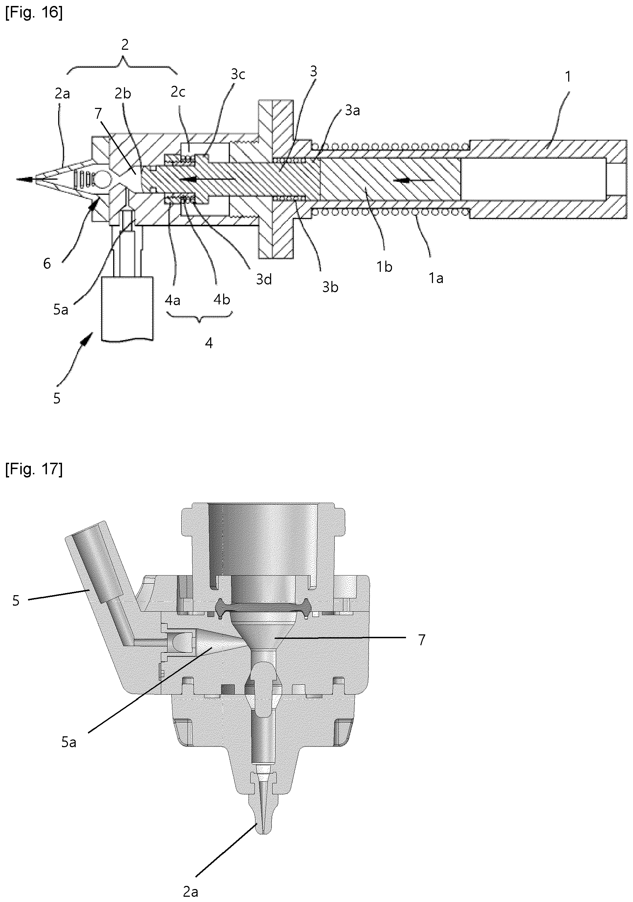

FIG. 16 is a diagram showing an embodiment of a needleless syringe disclosed in Patent Document 1 and FIG. 17 is a partial enlarged view showing the detailed structure of a nozzle that is applied to the needleless syringe disclosed in FIG. 16.

As shown in FIG. 16, the needleless syringe disclosed in Patent Document 1 is provided with one solenoid coil 1a, and includes a current supply unit (not shown) that causes the piston 3 to move forward by repeatedly supplying and cutting off a current to a solenoid coil 1a at a preset cycle, and an elastic member that applies an elastic force in a backward movement direction of the piston 3 when the current supply from the current supply unit (not shown) is cut off.

Specifically, when a movable magnetic body 1b advances a predetermined distance by a magnetic force generated in the solenoid coil 1a, it collides with a piston head 3a at the rear end of the piston 3, and the piston 3 advances by an impact force at this time. Further, springs 4a and 4b are compressed by the advance of the piston 3, and when the current supply to the solenoid coil 1a is stopped, the piston 3 retracts by the restoring force of the springs 4a and 4b.

Meanwhile, a diaphragm 2b is provided in front of the piston 3, and as the piston 3 retracts, the diaphragm 2b deforms, whereby the pressure in the valve chamber 7 is decreased and a drug solution is filled through a drug supply passage 5a extending into the valve chamber 7 from a drug solution charger 5 due to a pressure difference between the inside and outside of the valve chamber 7. Further, as the piston 3 advances, the diaphragm 2b applies instantaneous high pressure to the drug solution in the valve chamber 7 while deforming such that the drug solution is ejected through a nozzle hole 2a of a nozzle 2.

In the case of a needleless syringe having such a structure, in order to supply a drug from an external drug solution charger 5 into the valve chamber 7, the internal structure of the nozzle 2 becomes complicated, so there is a problem that mass production is difficult and the manufacturing costs increase. Further, due to this complicated structure, there is a concern that variations in product performance may occur. In addition, under a valve structure using a diaphragm, there is a problem that a large amount of drug remains in the nozzle without being ejected. In addition, since the coupling structure between the nozzle 2 and the drug solution charger 5 is complicated, there is a problem that it is difficult to design the nozzle or the drug solution charger into a cartridge type filled with drug so that a user at home can freely detachably couple the drug solution charger 5 or the nozzle 2 coupled with the drug solution charger 5 to the needleless syringe.

The present disclosure has been made in an effort to solve the problems described above and an objective of the present disclosure is to provide a needleless syringe that eliminates the complexity and instability of the nozzle structure of existing needleless syringes, reduces the manufacturing costs of needleless syringes including a nozzle, and enables a drug solution charger and a nozzle to be detachably coupled to a main body in the form of an integrated cartridge.

### [Technical Solution]

In order to achieve the objectives of the present disclosure, a needleless syringe according to the present disclosure is a needleless syringe including a driving unit, a conversion unit, and a nozzle unit, wherein the driving unit provides a pulse pressure or a pulse-type driving force for reciprocating a power transmission unit accommodated in the housing; the conversion unit converts the pulse pressure or a reciprocating motion of the power transmission unit into an incremental advancing motion of the piston such that, for each pulse of the pulse pressure or each reciprocating motion of the power transmission unit, the piston that has received kinetic energy from the driving unit incrementally advances by a predetermined distance toward the nozzle unit; and the nozzle unit has a drug reservoir containing a drug therein, and discharges the drug contained in the drug chamber in the form of a microjet through a nozzle hole in accordance with pressure applied to the drug by the piston.

Preferably, the piston may receive the kinetic energy by receiving the pulse-type pressure or by colliding with the power transmission unit; and the conversion unit may ensure that the kinetic energy transmitted to the piston is constant for each pulse or each reciprocating motion of the power transmission unit by advancing the driving unit by a predetermined distance each time the piston advances by the predetermined distance due to each pulse or one reciprocating motion of the power transmission unit.

Preferably, the piston may receive the kinetic energy by receiving the pulse-type pressure or by colliding with the power transmission unit; and the conversion unit may ensure that the kinetic energy transmitted to the piston is constant for each pulse or each reciprocating motion of the power transmission unit by increasing the magnitude of the pulse pressure or a driving force that is applied to the power transmission unit each time the piston advances by the predetermined distance due to each pulse or one reciprocating motion of the power transmission unit.

Preferably, the power transmission unit may be a plunger accommodated in the housing; the housing may have an internal chamber in a hollow internal space of the housing, and parts of the plunger and the piston may be accommodated in the internal chamber; a blocker may be provided at a nozzle unit-side front end of the housing to limit an advancing motion of the piston that advances toward the nozzle unit by collision with the plunger; and the needleless syringe may further include a displacing unit capable of advancing the housing and the blocker at least toward the nozzle unit and a controller configured to control the displacing unit.

Preferably, the power transmission unit may be a plunger accommodated in the housing; the housing may have an internal chamber in a hollow internal space of the housing, and parts of the plunger and the piston may be accommodated in the internal chamber; a blocker may be provided at a nozzle unit-side front end of the housing to limit an advancing motion of the piston that advances toward the nozzle unit by collision with the plunger; and the needleless syringe may further include a controller configured to control a driving force generated by the driving unit so that kinetic energy that is transmitted from the plunger to the piston is constant each time the plunger reciprocates.

Preferably, the nozzle unit may be configured to be detachable coupled to a main body of the needleless syringe including at least the housing and the blocker; and the nozzle unit-side end of the piston may extend into a chamber constituting the drug reservoir of the nozzle unit.

Preferably, the chamber of the nozzle unit may be configured such that one surface facing the main body of the needleless syringe is open to the outside; and a first cover may be detachably provided on the nozzle unit to be able to cover the open surface of the chamber when the nozzle unit is separated from the main body of the needleless syringe.

Preferably, a second cover made of a material that can be cut by the end of the piston when the end advances may be further provided on an inner side of the first cover facing the chamber; and a nozzle unit-side plunger configured to pressurize a drug contained in the chamber may be further provided inside the second cover.

Preferably, the driving unit may include a solenoid coil wound around an outer periphery of the internal chamber of the housing; and the controller may be configured to provide a pulse reciprocating motion of the plunger by controlling a current that is applied to the solenoid coil.

Preferably, the needleless syringe may further include: a diaphragm configured to deform with the pulse pressure or a reciprocating motion of the power transmission unit; a conversion unit-side chamber of which an internal pressure increases or decreases with deformation of the diaphragm; a driving fluid supply unit being in fluid communication with the conversion unit-side chamber through a driving fluid supply passage and configured to supply a driving fluid to the conversion unit-side chamber as the internal pressure of the conversion unit-side chamber increases or decreases with deformation of the diaphragm; and a check valve configured to allow only unidirectional flow of the driving fluid from the conversion unit-side chamber toward the internal chamber of the housing, wherein the piston may be configured to incrementally advance toward the nozzle unit by a hydraulic pressure of the driving fluid that is supplied from the driving fluid supply unit to the internal chamber of the housing.

Preferably, the driving unit may further include: a compressed gas tank configured to supply a compressed gas into the internal chamber of the housing through an inflow passage; and a valve provided on the inflow passage and configured to control inflow and outflow of the compressed gas into and from the internal chamber, and the driving unit may be controlled by the controller to provide the pulse pressure.

Preferably, the driving unit may further include: a compressed gas tank configured to supply a compressed gas into the internal chamber of the housing through an inflow passage; and a valve provided on the inflow passage and configured to control inflow and outflow of the compressed gas into and from the internal chamber, and a discharge passage configured to discharge a compressed gas flowing inside through the inflow passage to the outside of the needleless syringe may branch from the inflow passage and the valve may adjust opening and closing of the inflow passage and the discharge passage; the internal chamber may have at least one through-hole positioned behind a point at which the plunger first collides with a piston head of the piston when the plunger advances due to pressure of the compressed gas; the internal chamber may be in fluid communication with a compression chamber outside the internal chamber through the through-hole; as the plunger advances, air discharged from the internal chamber through the through-hole may be compressed in the compression chamber; when the discharge passage is opened by the valve and the compressed gas is discharged to the outside of the needleless syringe after the plunger collides with the piston, the air compressed in the compression chamber may flow back into the internal chamber through the through-hole due to a pressure difference between pressure of the internal chamber and pressure of the compression chamber; and the plunger may be moved to an original retracted position, from which the plunger started advancing, by the air flowing back into the internal chamber.

Preferably, the needleless syringe may further include a holding member configured to hold the plunger at the retracted position, wherein the holding member may wherein the holding member may hold the plunger at the retracted position until the plunger is advanced by the pressure of compressed air.

Preferably, the needleless syringe may include: a rod having a first end facing a piston head of the piston; an elastic member having a first end in contact with a second end of the rod and having a second end fixed to a main body of the needleless syringe; and a rotating member configured to rotate by a predetermined angle by a driving force generated by the driving unit, and having a first end rotatably connected to the rod, wherein when the rotating member is rotated by the driving force in a direction in which the second end of the rod compresses the elastic member, and then the driving force is removed, the first end of the rod may strike the piston head while the rod advances by an elastic force of the elastic member, thereby incrementally advancing the piston.

Preferably, the needleless syringe may further include: a compressed air chamber configured to supply compressed air into the internal chamber of the housing in the form of the pulse pressure; an air-compressing piston configured to compress air in the compressed air chamber through forward and backward reciprocating motions; a rotating member configured to rotate by a predetermined angle by a driving force generated by the driving unit, and having a first end rotatably connected to the air-compressing piston; and a valve configured to control inflow and outflow of the compressed air into and from the internal chamber, wherein, as the rotating member rotates, the air-compressing piston may compress air in the compressed air chamber while repeatedly reciprocating forward and backward, and flow of compressed air from the compressed air chamber toward the piston may be controlled by opening and closing the valve, whereby the piston may be incrementally advanced by pressure of the compressed air.

Preferably, the needleless syringe may further include an encoder capable of detecting a real-time position of the piston, wherein information on a stroke distance and a speed of the piston required to satisfy target injection amount and injection speed of a drug that is discharged from the nozzle unit may be stored in advance in the controller; the controller may calculate control signal values required to control the driving unit and the displacing unit to obtain the target injection amount and the injection speed of the drug, on the basis of real-time position information of the piston transmitted from the encoder and the information on the stroke distance and the speed of the piston stored in advance; and the controller may control the driving unit and the displacing unit on the basis of the calculated control signal values.

Preferably, the needleless syringe may further include an encoder capable of detecting a real-time position of the piston, wherein information on a stroke distance and a speed of the piston required to satisfy target injection amount and injection speed of a drug that is discharged from the nozzle unit may be stored in advance in the controller; the controller may calculate a control signal value required to control the driving unit to obtain the target injection amount and the injection speed of the drug, on the basis of real-time position information of the piston transmitted from the encoder and the information on the stroke distance and the speed of the piston stored in advance; and the controller may control the driving unit on the basis of the calculated control signal value.

### [Advantageous Effects]

According to the structure of the present disclosure, the drug in the nozzle can be ejected to the outside in a predetermined amount with an incremental advancing motion of the piston, so the nozzle itself can be used as a cartridge without the need to provide a separate drug charger outside the nozzle and to periodically supply the drug from the drug charger into the nozzle. Therefore, the existing complex drug charging mechanism can be simplified. As a result, the manufacturing costs of a nozzle and a needleless syringe including the nozzle can be reduced.

Further, according to the structure the present disclosure described above, the drug in the nozzle can be incrementally ejected multiple times per second through the piston's incremental advancing motion repeated multiple times per second, whereby it is possible to reduce the problem that a large amount of drug remains inside the nozzle, unlike existing needleless syringes in which drug charging and ejection alternately occur through the reciprocating motion of a plunger.

Moreover, according to the present disclosure, when the piston completes advancing, a user can separate and discard a nozzle-integrated cartridge from the main body of the needleless syringe and can easily mount a new cartridge onto the main body. Therefore, even at home, a user can easily perform cartridge removal and replacement.

Furthermore, according to the present disclosure, by changing the relative position of the blocker and the piston, the striking distance of the piston can be controlled, whereby it is possible to easily control the ejection speed and ejection amount of drug.

### [Description of Drawings]

FIG. 1 is a configuration diagram of a needleless syringe according to the present disclosure.
FIG. 2 is a diagram illustrating a first example of a mechanism for converting a pulse motion into an incremental advancing motion in the needleless syringe according to the present disclosure.
FIG. 3 is a diagram illustrating a second example of the mechanism for converting a pulse motion into an incremental advancing motion in the needleless syringe according to the present disclosure.
FIG. 4 is a cross-sectional perspective view of a needleless syringe according to an exemplary embodiment of the present disclosure.
FIGS. 5a to 5g are diagrams illustrating, step by step, a drug ejection process using the needleless syringe shown in FIG. 4.
FIG. 6 is a perspective view of the needleless syringe shown in FIG. 4, viewed from a direction different from that of FIG. 3.
FIG. 7 is a partial enlarged view for explaining a coupling structure between a nozzle unit and a conversion unit of the needleless syringe shown in FIG. 4.
FIGS. 8a and 8b are cross-sectional perspective views of a nozzle unit that is used in the needleless syringe shown in FIG. 4.
FIG. 9 is a cross-sectional view for explaining a nozzle unit and a conversion unit of a needleless syringe according to another exemplary embodiment of the present disclosure.
FIG. 10 is a configuration diagram of a needleless syringe according to an exemplary embodiment of the present disclosure using compressed air.
FIG. 11 is a configuration diagram of a needleless syringe according to another exemplary embodiment of the present disclosure using compressed air.
FIG. 12 is a configuration diagram of a needleless syringe according to another exemplary embodiment of the present disclosure using an elastic member and a motor.
FIG. 13 is a configuration diagram of a needleless syringe according to another exemplary embodiment of the present disclosure using compressed air and a motor.
FIG. 14 is a diagram for explaining the position and speed control of a piston according to an exemplary embodiment of the present disclosure.
FIG. 15 is a diagram for explaining PID control that is used for position and speed control of the piston shown in FIG. 14.
FIG. 16 is a configuration diagram of a needleless syringe of the related art.
FIG. 17 is a partial enlarged view of a nozzle unit of a needleless syringe of the related art.

**Reference numerals indicate the following.**

| | | | |
|---|---|---|---|
| 1: | body | 1a: | solenoid coil |
| 2a: | nozzle unit | 2b: | diaphragm |
| 2c: | main hole | 3: | piston |
| 3a: | piston head | 3b: | spring |
| 3c: | flange | 4a: | fixing blocker |
| 4b: | length adjustment blocker | 5: | drug charger |
| 5a: | drug supply hole | 6: | nozzle unit opening/closing valve |
| 7: | valve chamber | 10: | driving unit |
| 10a: | housing | 10b: | housing internal chamber |
| 11: | solenoid coil | 12: | driving unit-side plunger |
| 13: | housing holder | 14: | step motor |
| 14a: | step motor holder | 15: | driving shaft |
| 16: | housing-side screw joint | 16a: | power switching mechanis |
| 20: | conversion unit | 21: | piston |
| 21a: | piston head | 21b: | piston end |
| 22a: | blocker | 22b: | blocker holder |
| 22c: | driving shaft support | 23: | nozzle unit holder |
| 23a: | female threaded portion | 24: | blocker-side screw joint |
| 24a: | power switching mechanism | 30: | nozzle unit |
| 31a: | nozzle unit front end | 31: | chamber |
| 32a: | nozzle unit-side plunger | 33: | nozzle hole |
| 34: | flange | 35: | male threaded portion |
| 36: | first cover | 37: | nozzle unit end |
| 37a: | nozzle unit end hole | 38: | second cover |
| 41: | ball pin | 42: | spring |
| 43: | conversion unit-side housing | 50: | driving fluid supply unit |
| 51: | driving fluid supply channel | 52: | conversion unit-side chamber |
| 60: | driving unit main body | 61: | compressed air hose |
| 63: | valve | 64: | trigger |
| 71: | piston head | 72: | elastic member |
| 73: | step motor | 74: | trigger |
| 75: | blocker | 76: | piston head locking portion |
| 80: | compressed air chamber | 81: | piston |
| 82: | communicating hole | 100: | drug |
| 101: | base | 110: | driving fluid |

### [Best Mode]

Hereinafter, exemplary embodiments of a needleless syringe according to the present disclosure are described in detail with reference to the accompanying drawings.

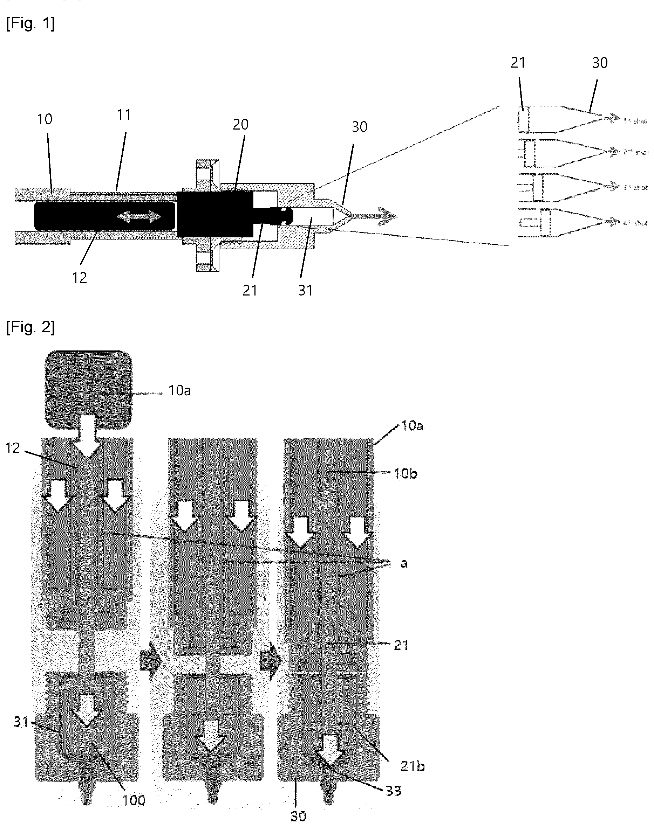

FIG. 1 is a configuration diagram of a needleless syringe according to the present disclosure. As shown in FIG. 1, a needleless syringe according to the present disclosure includes a driving unit 10, a conversion unit 20, and a nozzle unit 30.

The driving unit 10 provides a pulse reciprocating motion of a driving-side plunger 12 provided inside the driving unit 10 using a driving force generated from a predetermined power source. In the example shown in FIG. 1, the driving unit 10 has a solenoid coil 11 on its outer periphery, and provides a pulse reciprocating motion of the driving-side plunger 12 by periodically changing the direction of the current that is applied to the solenoid coil 11. However, the present disclosure is not limited thereto, and any structure capable of providing a reciprocating motion of the driving-side plunger 12 in the driving unit 10 can be applied. In FIG. 1, the pulse reciprocating motion of the plunger 12, which is a power transmission unit, is disclosed, but the present disclosure is not limited to this embodiment. As described below, the driving unit 10 can provide pulse-type pressure, such as compressed air.

The conversion unit 20 is configured to convert the pulse pressure provided from the driving unit 10 or the pulse reciprocating motion of the driving-side plunger 12 into an incremental advancing motion of a nozzle unit-side plunger 21 or a piston. Here, the incremental advancing motion refers to a motion in which a pressurizing unit (the nozzle unit-side plunger 21 or the piston) for a drug 100 contained in a chamber 31 of a nozzle unit 30 advances step by step by a predetermined distance for each pulse of the pulse pressure provided from the driving unit 10 or for each reciprocation of the pulse reciprocating motion. Further, the drug 100 fills a drug-containing space formed by the chamber 31 and the pressurizing unit described above when it is initially charged into the chamber 31. Accordingly, each time the pressurizing unit described above advances step by step, a drug is ejected in the form of a microjet with a pressure and a predetermined injection volume sufficient to penetrate the skin and reach target tissue within the skin of a patient from a nozzle hole 33 of the nozzle unit 30. According to this structure, it is possible to discharge the drug 100 precharged in the nozzle unit 30 in a predetermined amount for each reciprocation of the pulse reciprocating motion, without the need for the drug to be charged from the external drug reservoir into the nozzle unit 30 for each pulse of the pulse pressure or for each reciprocation of the pulse reciprocating motion.

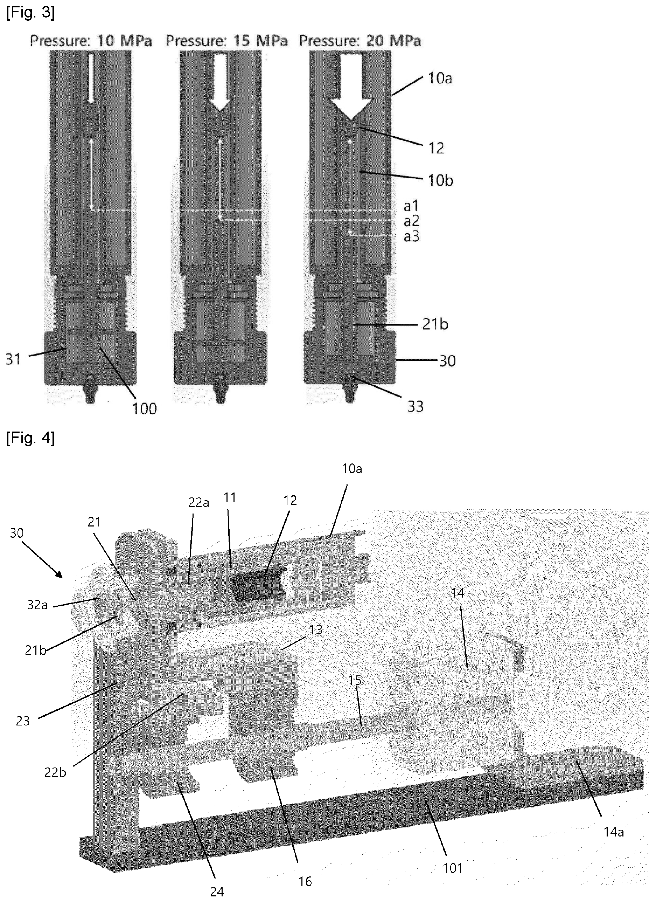

FIGS. 2 and 3 illustrate different mechanisms for converting pulse pressure or a pulse motion into an incremental advancing motion in a needleless syringe according to the present disclosure.

In the mechanism shown in Figure 2, for example, a piston 21 for pressurizing the drug 100 in the chamber 31 of the nozzle unit 30 and a plunger 12 for pressing the piston 21 toward the nozzle unit 30 are accommodated in an internal chamber 10b of a hollow housing 10a. A piston end 21b of the piston 21 facing the nozzle unit 30 extends into the chamber 31 of the nozzle unit 30. The chamber 31 of the nozzle unit 30 and the piston end 21b form a drug-containing space, and the inside of the drug-accommodating space is filled with the drug 100. Preferably, in order to apply a predetermined pressure to the drug 100 in the drug-containing space, the piston end 21b may have a disc shape with a diameter substantially the same as the inner diameter of the opening of the chamber 31.

Further, as described above, the drive unit 10 generates a power source that allows the plunger 12 to reciprocate in the longitudinal direction of the internal chamber 10b. Preferably, as the power source generated by the drive unit 10, the solenoid coil described above or a combination of compressed air and an elastic member may be used. In this case, the plunger 12 can be advanced using an electromagnetic force generated by the solenoid coil or the pressure of the compressed air, and when the plunger 12 has advanced a predetermined distance or more, it can be retracted using the elastic force of the elastic member.

Meanwhile, when the plunger 12 advances toward the piston 21 due to the power generated by the drive unit 10 and collides with the piston 21, kinetic energy is transmitted from the plunger 12 to the piston 21, whereby the piston 21 pressurizes the drug 100 in the chamber 31 while advancing a predetermined distance toward the nozzle unit 30. The drug 100 is ejected in a predetermined amount from the nozzle hole 33, which is in fluid communication with the chamber 31, by the pressure applied by the piston 21. Further, the plunger 12, which has collided with the piston 21, is retracted by a driving force provided from the drive unit 10.

Meanwhile, in the mechanism described in Figure 2, as shown in Figure 2, after the forward stroke of one reciprocation of the plunger 12, a housing 10a including the plunger 12 is advanced by a distance equal to the distance the piston 21 has advanced, using an actuator or the like, which is not shown. That is, the drive unit including the plunger 12 is advanced by the same distance as the distance the piston 21 has advanced. In this case, as shown in FIG. 2, even when the piston 21 advances a predetermined distance through one reciprocating motion of the plunger 12, the driving unit including the plunger 12 also advances by the same distance. Therefore, the relative position between the plunger 12 and the piston 21 in the housing 10a and the position (a) where the plunger 12 collides with the piston 21 are the same as those before one reciprocating motion. Therefore, when a driving force of the same magnitude as that of the previous reciprocating motion is applied to the plunger 12, the piston 21 can be advanced by the same distance as that of the previous reciprocating motion. That is, by reciprocating the plunger 12 with the same driving force, an incremental advancing motion that advances the piston 21 step by step can be implemented. Therefore, it becomes possible to eject a predetermined amount of the drug 100 contained in the chamber 31 of the nozzle unit 30 for each reciprocating motion. Further, when the piston 21 advances a predetermined distance or more in the chamber 31 as a result of multiple incremental advancing motions, the ejection of the contained drug 100 is completed, and a user can easily replace the nozzle unit 30 by separating it from the housing 10a.

In FIG. 3, the reciprocating motion of the plunger 12 is converted into the incremental advancing motion of the piston 21 through a mechanism different from the mechanism shown in FIG. 2. In the mechanism illustrated in FIG. 2, by advancing the driving unit by the same distance each time the piston 21 advances a predetermined distance, the collision position of the piston 21 and the plunger 12 is maintained equally in the housing 10a, whereby the reciprocating motion of the plunger 12 is converted into the incremental advancing motion of the piston 21. On the other hand, in FIG. 3, the position of the housing 10a is not changed, and each time the piston 21 advances a predetermined distance, the magnitude of the driving force applied to the plunger 12 or the magnitude of the pulse pressure is varied. That is, in FIG. 3, since the position of the housing 10a is not changed, unlike the mechanism shown in FIG. 2, the collision positions (a1, a2, a3) of the piston 21 and the plunger 12 in the housing 10a vary for each reciprocation of the plunger 12. In this case, if the magnitude of the driving force applied to the plunger 12 is maintained constant for each reciprocation, as in the mechanism shown in FIG. 2, the magnitude of the kinetic energy that is applied to the piston 21 varies for each reciprocation of the plunger 12. Therefore, since the distance by which the piston 21 advances varies for each reciprocation of the plunger 12, the ejection amount of the drug 100 that is ejected from the nozzle hole 33 of the nozzle unit 30 also varies for each reciprocation of the plunger 12. Therefore, it becomes impossible to inject a predetermined amount of the drug 100 to an accurate depth for each ejection.

Therefore, in the mechanism shown in FIG. 3, the driving force that is applied to the plunger 12 is varied for each reciprocation of the plunger 12. For example, in the example shown in FIG. 3, if the pressure that is applied to the plunger 12 during a first reciprocating motion is 10 MPa, a higher pressure of 15 MPa is applied to the plunger 12 during a second reciprocating motion, and then a pressure of 20 MPa higher than that during the second reciprocating motion is applied during a third reciprocating motion. The magnitude of the pressure that is applied to the plunger 12 for each reciprocating motion of the plunger 12 is determined as a value at which the kinetic energy that is transmitted from the plunger 12 to the piston 21 for each reciprocating motion becomes constant. The magnitude of such pressure can be calculated by measuring the current position of the piston 21 using an encoder, etc. to be described below. Further, the magnitude of pressure can be controlled, for example, by controlling the magnitude of the current that is applied to the solenoid coil or by varying the air pressure of the compressed air that is ejected toward the plunger 12.

Hereinafter, a specific embodiment of a needleless syringe that implements an incremental advancing motion from the reciprocating motion generated by the driving unit 10 in accordance with each of the mechanisms described above are described in detail with reference to the accompanying drawings.

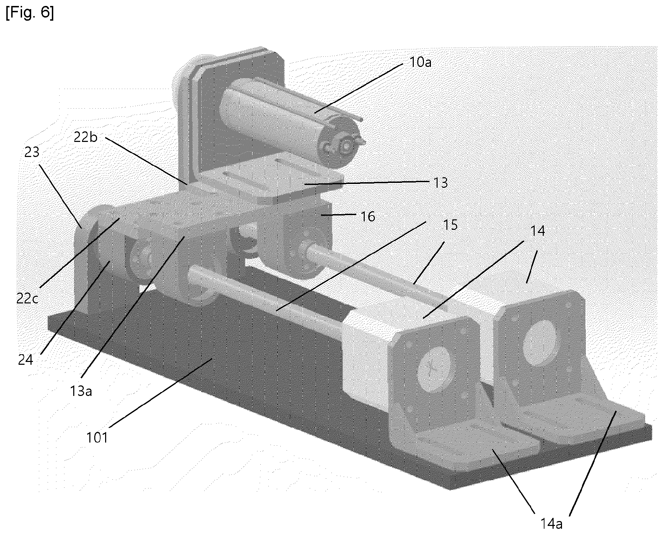

FIG. 4 is a cross-sectional perspective view of a needleless syringe according to an exemplary embodiment of the present disclosure and FIG. 6 is a perspective view of the needleless syringe shown in FIG. 4, viewed from a direction different from that of FIG. 4.

In the example shown in FIG. 4, the driving unit 10 includes the housing 10a forming the body, the driving unit-side plunger 12, which is, for example, a magnetic body constituting the core of a solenoid actuator, is accommodated in the internal chamber 10b of the housing 10a, and a solenoid coil 11 is wound on the outer periphery of the internal chamber 10b.

Further, a housing support 13 for supporting the housing 10a is formed at the housing 10a. Preferably, the housing support 13 is integrally formed at a nozzle unit 30-side end of the housing 10a and extends downward from the housing 10a. The housing support 13 is connected to a housing-side screw joint 16, so when the driving shaft 15 is rotated by a step motor 14, the housing 10a can be moved forward or backward by a predetermined distance in accordance with the rotation direction and rotation amount of the driving shaft 15, as described below.

In the example illustrated in FIG. 4, the conversion unit 20 includes a piston 21 and a blocker 22a. The piston 21 includes a rod-shaped main body, a disc-shaped piston head 21a provided at a driving unit-side rear end of the main body, and a disc-shaped piston end 21b provided at a nozzle unit-side front end of the main body. The rod-shaped main body of the piston 21 extends in the longitudinal direction such that the piston head 21a is positioned inside the internal chamber 10b of the housing 10a and the piston end 21b is positioned inside the chamber 31 of the nozzle unit 30 to be described below.

The blocker 22a includes a member having a hollow shape through which the rod-shaped main body of the piston 21 passes, and the driving unit-side end of the member having a hollow shape faces the piston head 21a of the piston 21, whereby a region that collides with the piston head 21a when the piston 21 advances is formed.

Further, a blocker holder 22b for holding the blocker 22a is formed at the blocker 22a. Preferably, the blocker holder 22b is integrally formed at a nozzle unit 30-side end of the member having a hollow shape of the blocker 22a and extends downward from the blocker 22a. The blocker holder 22a is connected to a blocker-side screw joint 24, so when the driving shaft 15 is rotated by a step motor 14, the blocker 22a can be moved forward or backward by a predetermined distance in accordance with the rotation direction and rotation amount of the driving shaft 15, as described below.

The nozzle unit 30 may be formed as a hollow cylindrical member capable of containing a drug therein. A nozzle unit-side plunger 32a is inserted in the chamber 31 of the nozzle unit 30 to be movable relative to the inner wall of the chamber 31 such that a drug-containing region and the other region are separated in the chamber 31 by the nozzle-unit-side plunger 32a. In the example shown in FIG. 4, the nozzle unit-side plunger 32a is configured separately from the piston end 21b of the piston 21, but the present disclosure is not limited to this configuration. For example, the nozzle unit-side plunger 32a may not be provided separately and may have the functions of both the piston end 21b and the nozzle unit-side plunger 32a. The nozzle unit 30 is detachably mounted to the needleless syringe main body composed of the conversion unit 20 and the driving unit 10. Preferably, a nozzle unit end 37, which is a conversion unit-side end of the nozzle unit 30, can be screwed into a nozzle unit holder 23 formed in the needleless syringe main body. To this end, a male threaded portion 35 may be formed on the nozzle unit end 37 and a female threaded portion 23a may be formed on the nozzle unit holder 23. In this case, a user can easily attach or detach the nozzle unit 30 simply by relatively rotating the nozzle unit 30 with respect to the nozzle unit holder 23. The specific structure of the nozzle unit 30 is described below.

A base 101 is a member for supporting the driving unit 10, the conversion unit 20, and the nozzle unit 30 that constitute the needleless syringe. A step motor holder 14a for fixing and supporting the step motor 14 is provided on the upper surface of the base 101 and can fix and support the lower end of the nozzle unit holder 23. The front end of the drive shaft 15 of the step motor 14 is preferably rotatably supported by a driving shaft support 22c, and in this configuration, the driving shaft support 22c may be integrally formed with the nozzle unit holder 23.

Meanwhile, a portion of the outer periphery of the driving shaft 15 may be connected to the housing-side screw joint 16 and the blocker-side screw joint 24 to be rotatable with respect to them through power switching mechanisms 16a and 24a, respectively. Preferably, the power switching mechanisms 16a and 24a perform the function of transmitting or blocking the rotational power of the driving shaft to the housing-side screw joint 16 and the blocker-side screw joint 24, respectively, through a known switching unit. When the rotational power of the driving shaft 15 is transmitted to the housing-side screw joint 16 and the blocker-side screw joint 24 by the power switching mechanisms 16a and 24a, the housing 10a or the blocker 22a can be moved forward or backward by a predetermined distance in accordance with the rotation direction and the rotation amount of the driving shaft 15, as described below.

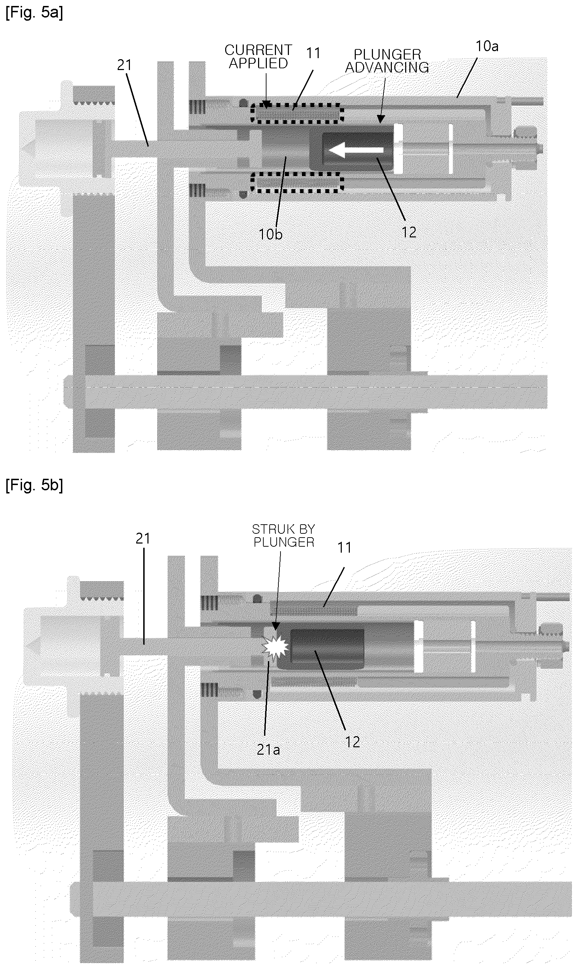

FIGS. 5a to 5g are diagrams illustrating, step by step, a drug ejection process using the needleless syringe shown in FIG. 4. Hereinafter, a drug ejection operation using the needleless syringe according to the present disclosure is described in detail with reference to the illustrations of FIGS. 5a to 5g.

First, as shown in FIG. 5a, a current is applied to the solenoid coil 11, whereby the driving unit-side plunger 12 starts advancing toward the piston 21.

Next, as shown in FIG. 5b, as the driving unit-side plunger 12 advances, the front end of the driving unit-side plunger collides with the piston head 21a of the piston 21.

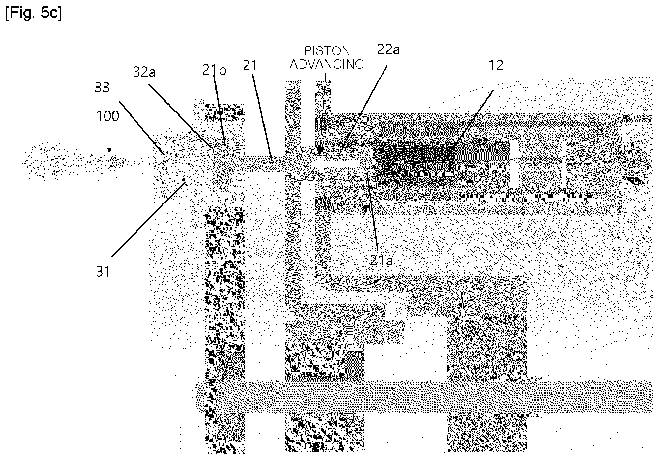

Next, as shown in FIG. 5c, the piston 21 advances due to the collision between the front end of the driving unit-side plunger 12 and the piston head 21a of the piston 21, and accordingly, the piston end 21b of the piston 21 presses and moves forward the adjacent nozzle unit-side plunger 32a. The drug 100 in the chamber 31 of the nozzle unit 30 is compressed by the distance that the nozzle unit-side plunger 32a advances, and the drug is ejected through the nozzle hole 33 of the nozzle unit 30 by a predetermined amount. Meanwhile, the advancing of the piston 21 stops when the piston head 21a collides with the blocker 22a, whereby the distance that the nozzle unit-side plunger 32a advances ultimately becomes the distance that the piston 21 advances from the time point at which the piston head 21a starts moving to the time point at which the piston head 21a collides with the blocker 22a.

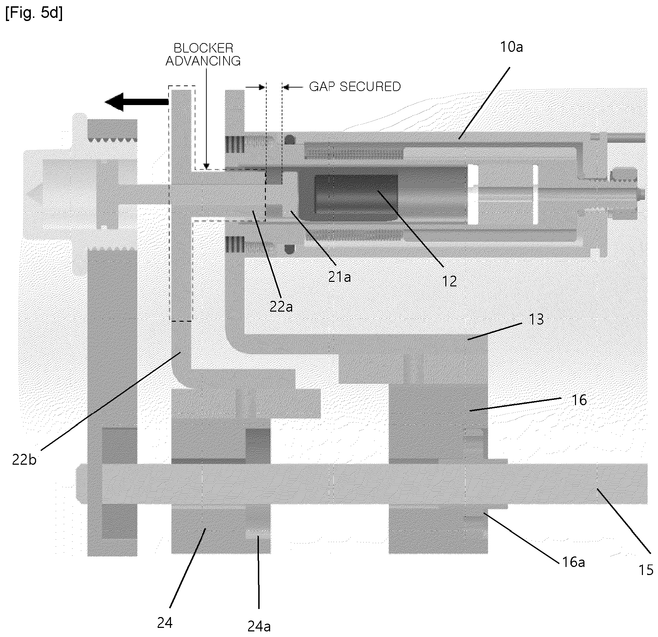

Next, as shown in FIG. 5d, the step motor 14 rotates the driving shaft 15, and the rotational force of the driving shaft 15 is transmitted to the blocker-side screw joint 24 through the power switching mechanism 24a, thereby advancing the blocker 22a by a predetermined distance. Meanwhile, the power switching mechanism 16a operates to block transmission of the rotational force of the driving shaft 15 to the housing-side screw joint 16, so even if the driving shaft 15 rotates, the housing 10a does not move and remains stationary. That is, in this state, only the blocker 22a advances and the housing 10a remains stationary. Therefore, as the blocker 22a advances, a predetermined gap is secured between the piston head 21a and the blocker 22a that were in contact with each other.

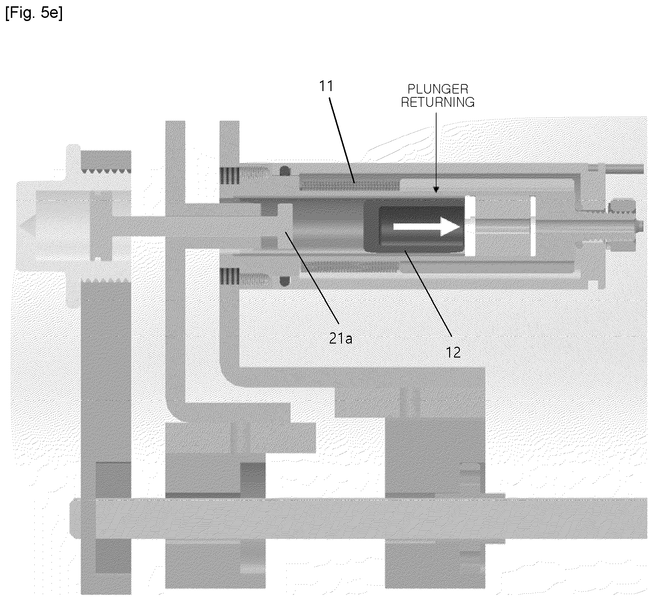

Next, as shown in FIG. 5e, a current flows through the solenoid coil 11 in the opposite direction to the previous direction, so the driving unit-side plunger 12 retracts.

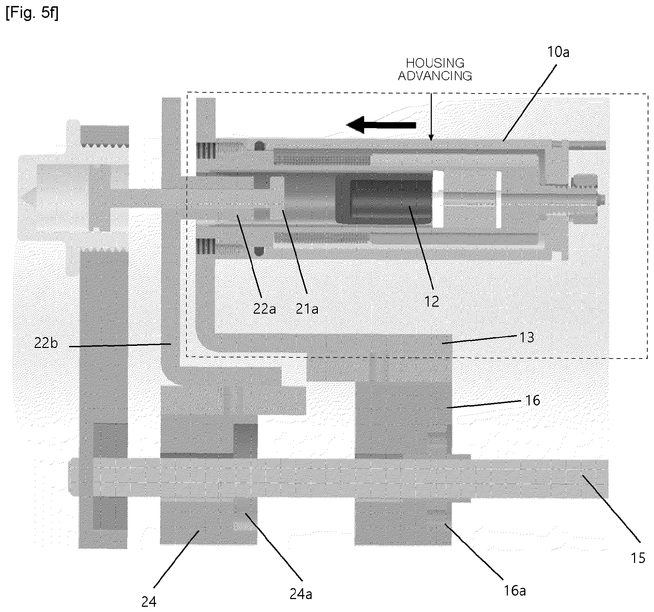

Next, as shown in FIG. 5f, the step motor 14 rotates the driving shaft 15, and the rotational force of the driving shaft 15 is transmitted to the housing-side screw joint 16a through the power switching mechanism 24a, thereby advancing the housing 10a including a coil module by a predetermined distance. Meanwhile, in this case, the power switching mechanism 24a operates to block transmission of the rotational force of the driving shaft 15 to the blocker-side screw joint 24, so even if the driving shaft 15 rotates, the blocker 22a does not move and remains stationary. That is, in this state, only the housing 10a advances and the blocker 22a remains stationary. Therefore, the distance between the blocker 22a and the housing 10a decreases, and the same distance as the distance between the piston head 21a of the piston 21 and the driving unit-side plunger 12 before the driving unit-side plunger 12 advances is maintained, as shown in FIG. 4a.

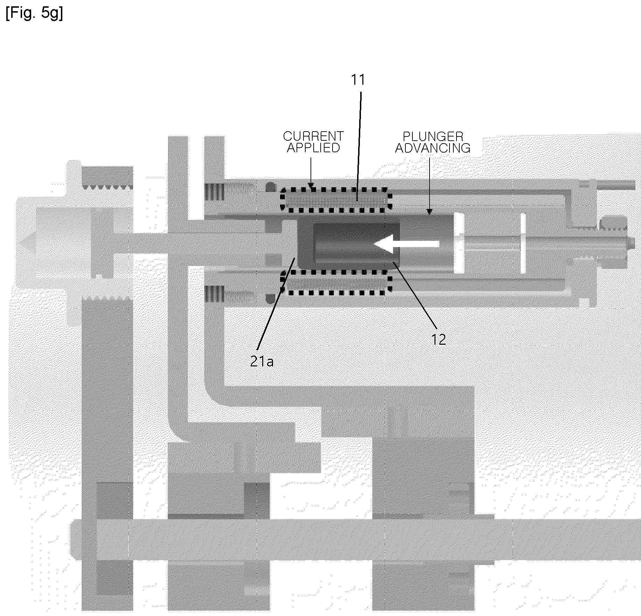

Next, as shown in FIG. 5g, similar to that shown in FIG. 4a, a current is applied to the solenoid coil 11, whereby the driving-unit-side plunger 12 is advanced toward the piston head 21a. By repeatedly performing the operations shown in FIGS. 5a to 5f, the driving unit-side plunger 12 performs a pulse reciprocating motion, and the nozzle unit-side plunger 32a advances by a predetermined distance during one reciprocation of the driving unit-side plunger 12. Therefore, while the driving unit-side plunger 12 performs multiple reciprocations, the nozzle unit-side plunger 32a can perform multiple stepwise advances.

In the embodiment shown in FIGS. 5a to 5g, when the driving shaft 15 is rotated by the step motor 14, the housing 10a and the blocker 22a are advanced individually, but the present disclosure is not limited to this embodiment. That is, instead of sequentially moving the housing 10a and the blocker 22a one by one, by changing the direction of the current that is applied to the solenoid coil 11 after the driving unit-side plunger 12 collides with the piston head 21a, the housing 10a and the blocker 22a may be moved together with the driving unit-side plunger 12 retracted and the position of the piston 21 maintained. Even in this case, as in the previous reciprocation of the driving unit-side plunger 12, the position of the piston 21 in the housing 10a can be maintained constant during the next reciprocation of the driving-unit-side plunger 12.

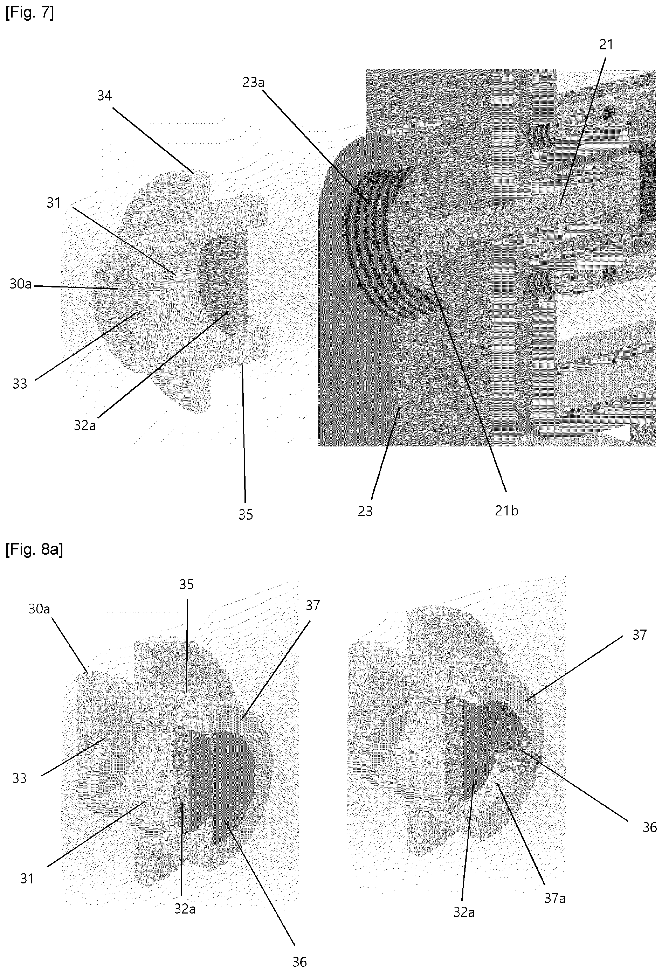

FIG. 7 is a partial enlarged view for explaining a coupling structure between a nozzle unit and a conversion unit of the needleless syringe shown in FIG. 4.

As shown in FIG. 7, the nozzle unit 30 may be formed as a hollow cylindrical member capable of containing a drug therein. The nozzle hole 33, which is a through-hole of a predetermined size, is formed at the nozzle-unit front end 30a that is the front end of the cylindrical member. The nozzle unit-side plunger 32a is inserted in the chamber 31 of the nozzle unit 30 to be movable relative to the inner wall of the chamber 31 such that a drug-containing region and the other region are separated in the chamber 31 by the nozzle-unit-side plunger 32a. The nozzle unit 30 is detachably mounted to the needleless syringe main body composed of the conversion unit 20 and the driving unit 10. Preferably, the nozzle unit end 37, which is a conversion unit-side end of the nozzle unit 30, can be screwed into the nozzle unit holder 23 formed in the needleless syringe main body. To this end, the male threaded portion 35 may be formed on the nozzle unit end 37 and the female threaded portion 23a may be formed on the nozzle unit holder 23. Meanwhile, a flange 34 may be formed integrally with the cylindrical member to surround the outer periphery of the hollow cylindrical member of the nozzle unit 30. In this case, when the nozzle unit 30 is coupled to the nozzle unit holder 23, one surface of the flange 34 is brought into close contact with the corresponding surface of the nozzle unit holder 23. Therefore, this structure prevents the drug 100 from leaking to the outside and allows the coupling strength when a user couples the nozzle unit 30 to the nozzle unit holder 23 to be adjusted within an appropriate range.

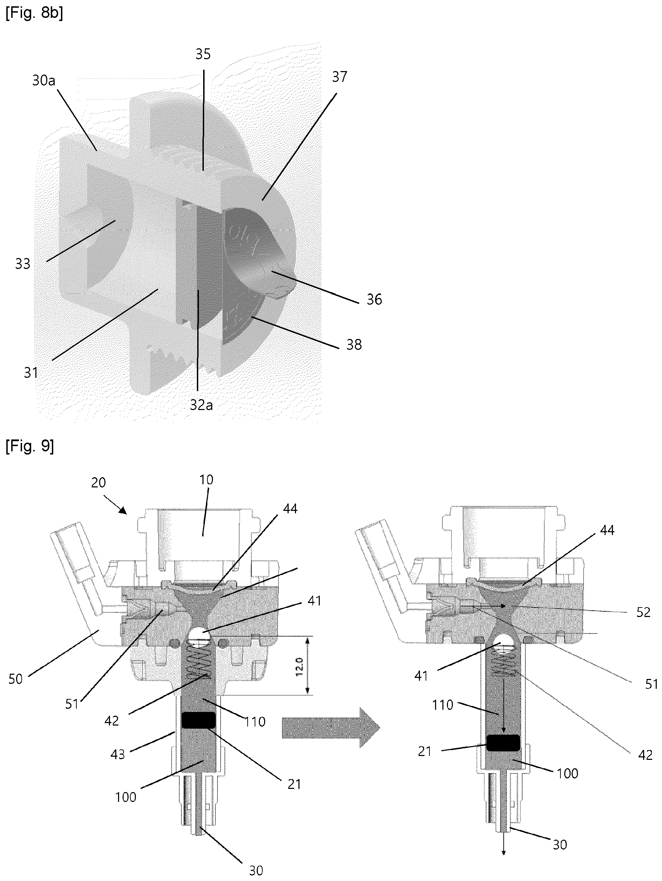

FIGS. 8a and 8b are cross-sectional perspective views of a nozzle unit that is used in the needleless syringe shown in FIG. 4.

As shown in FIG. 8a, before the nozzle unit 30 is coupled to the main body of the needleless syringe, a nozzle unit end hole 37a on the nozzle unit end 37, which is coupled to the main body of the needleless syringe, is detachably covered with a first cover 36. Therefore, a user can manually remove the first cover 36 as shown in FIG. 8a and then couple the nozzle unit 30 to the main body of the needleless syringe before coupling the nozzle unit 30 to the main body of the needleless syringe. According to this configuration, the first cover 36 can prevent the drug contained in the chamber 31 of the nozzle unit 30 from leaking to the outside during delivery or before coupling to the main body for use.

In FIG. 8b, in addition to the example shown in FIG. 8a, a second cover 38 may be provided inside the first cover 36 such that the second cover 38 covers the nozzle unit end hole 37a at the nozzle unit end 37 and the first cover 36 covers the second cover 38. In this case, before coupling the nozzle unit 30 to the main body of the needleless syringe, a user manually removes the first cover 36 as shown in FIG. 8a and then couples the nozzle unit 30 to the main body, during which the piston end 21b passes through the second cover 38 and comes into contact with the nozzle unit-side plunger 32a. In the case of employing such a structure, in the process of coupling the nozzle unit 30 to the main body of the needleless syringe after manually removing the first cover 36, it is possible to prevent the drug 100 from leaking between the nozzle unit-side plunger 32a and the inner wall of the chamber 31 of the nozzle unit 30.

FIG. 9 is a cross-sectional view for explaining a nozzle unit and a conversion unit of a needleless syringe according to another exemplary embodiment of the present disclosure.

Compared with the embodiment described with reference to FIG. 4, the needleless syringe according to the preferred embodiment shown in FIG. 9 differs in that the conversion unit 20 employs a driving fluid 110 to convert the pulse reciprocating motion of the driving unit 10 into the stepwise advancing motion of the piston 21.

The driving fluid 110 is stored in a driving fluid supply unit 50, and the driving fluid supply unit 50 is in fluid communication with a conversion unit-side chamber 52 inside a conversion unit-side housing 43 of the conversion unit 20 through the driving fluid supply passage 51. Meanwhile, a diaphragm 44 is provided at the joint of the driving unit 10 and the conversion unit-side chamber 52. Therefore, when the plunger of the driving unit 10, for example as shown in FIG. 1, reciprocates due to the driving of a solenoid actuator, the diaphragm 44 deforms accordingly, whereby the pressure inside the conversion unit-side chamber 52 increases or decreases. Specifically, when the plunger of the driving unit 10 retracts, the diaphragm 44 bends in the direction opposite to the conversion unit-side chamber 52, thereby decreasing the pressure inside the conversion unit-side chamber 52. Accordingly, a pressure difference occurs between the inside of the driving fluid supply unit 50 and the conversion unit-side chamber 52, so the driving fluid flows into the conversion unit-side chamber 52. In the embodiment shown in FIG. 9, the diaphragm 44 is deformed by reciprocating movement of the plunger, but the present disclosure is not limited to this embodiment. For example, the diaphragm 44 may be deformed by a pulse-type pressure, such as compressed air, which is applied to the diaphragm 44.

Next, when the plunger of the driving unit 10 advances, the diaphragm 44 bends toward the conversion unit-side chamber 52, thereby increasing the pressure inside the conversion unit-side chamber 52. This pressure acts on the piston 21 of the nozzle unit 30 through the driving fluid 110, and as the piston 21 advances, it applies pressure to the drug 100 inside the nozzle unit 30, thereby causing the drug 100 to be ejected at high pressure through the nozzle hole of the nozzle unit 30.

Meanwhile, a check valve, for example, which is composed of a ball pin 41 and a spring 42, is provided inside the conversion unit-side chamber 52 and prevents the driving fluid inside the conversion unit-side chamber 52 from flowing backward. Therefore, when the plunger of the driving unit 10 retracts, only flow of the driving fluid from the driving fluid supply unit 50 toward the inside of the conversion unit-side chamber 52 is formed, and the driving fluid on the downstream side of the check valve cannot move to the space on the upstream side of the check valve. Therefore, during reciprocation of the plunger of the driving unit 10, the driving fluid is continuously supplied from the driving fluid supply unit 50 into the conversion unit-side chamber 52. Therefore, according to this configuration, as in the embodiment shown in FIG. 3, during reciprocation of the plunger of the driving unit 10, the stepwise forward driving of the piston 21 in the nozzle unit 30 can be implemented by the amount of fluid supplied into the nozzle unit 30 through the conversion unit-side chamber 52.

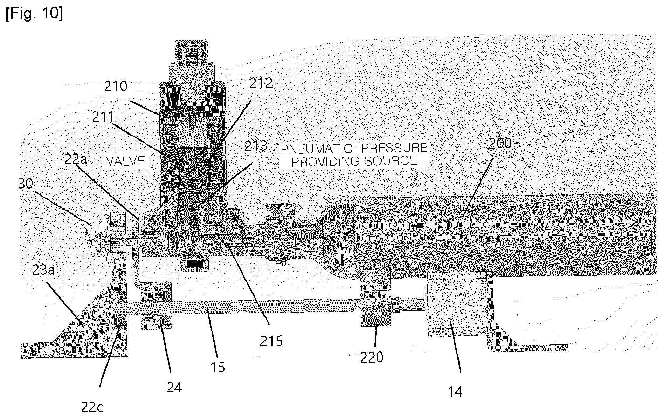

In the embodiment shown in FIG. 4, a solenoid actuator is applied as a driving source of the driving unit 10, but the present disclosure is not limited to this feature. For example, as shown in FIG. 10, the piston may be driven using compressed air and an elastic member. Meanwhile, as described above, in the embodiment shown in FIG. 4, a structure that moves the housing 10a forward is employed to implement a mechanism that converts a reciprocating motion into an incremental advancing motion. However, the present disclosure is not limited to the above embodiment. For example, instead of employing a structure that moves the housing 10a to convert a reciprocating motion into an incremental advancing motion, the current value that is applied to the solenoid coil 11 may be controlled for each reciprocation of the driving unit-side plunger 12 to control the force that is applied to the driving unit-side plunger 12. In this case, as described above with reference to FIG. 3, the kinetic energy that is transmitted to the piston 21 through the driving unit-side plunger 12 can be made identical for each reciprocating motion of the driving unit-side plunger 12. Therefore, even without moving the housing 10a and the blocker 22a, the nozzle unit-side plunger 32a can be caused to advance a predetermined distance step by step each time the driving unit-side plunger 12 reciprocates.

The embodiment shown in FIG. 10 is identical to the example shown in FIG. 4, except for the power generation structure of the driving unit 10. Therefore, the same reference numerals are assigned to structures identical to those in the embodiment shown in FIG. 4, and redundant descriptions are omitted.

In the example shown in FIG. 10, a compressed gas is used as a means for applying pressure to the piston 21. The compressed gas, which is a source providing pneumatic pressure, is stored in a compressed gas tank 200, and the pressure pulses generated by the compressed gas are regulated through a valve 210 provided on the upstream side of the compressed gas tank 200.

The valve 210 includes, for example, a core 212 formed as a magnetic body therein and includes a solenoid coil 211 provided to surround the core 212.

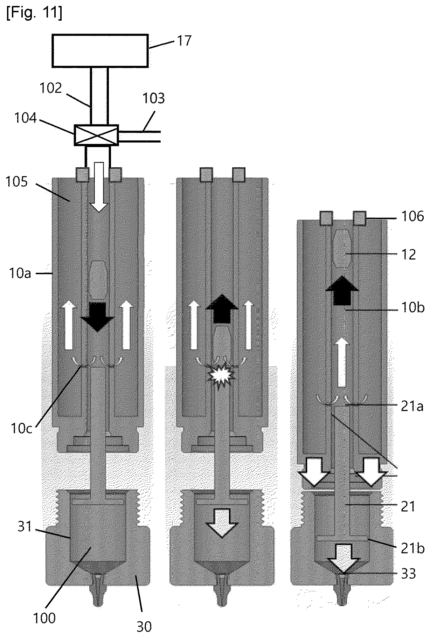

A valve channel 215 of which an end is in fluid communication with the compressed gas tank 200 and that accommodates the piston head 21a is formed in the valve 210, and a plunger 213 that can open and close the valve passage 215 is formed at an end of the core 212. Therefore, depending on the direction of the current that is applied to the solenoid coil 211 of the valve 210, the core 212 moves in the vertical direction, whereby the plunger 213 can open and close the valve passage 215. When a current is applied to the solenoid coil 211 in the direction in which the core 212 ascends, the compressed air stored in the compressed gas tank 200 is instantaneously supplied through the valve channel 215, thereby pressing the piston 21 forward and moving the piston 21 forward. Further, at the time point when the piston 21 reaches the top dead center and collides with the blocker 22a, a current flows in the solenoid coil 211 in the direction in which the core 212 descends, whereby the flow of the compressed gas passing through the valve passage 215 is blocked. Meanwhile, similar to the housing 10a shown in FIG. 3, a compressed gas tank-side screw joint 220 is provided under the compressed gas tank 200, and when the driving shaft 15 is rotated by the step motor 14, the compressed gas tank 200 advances accordingly, similar to the housing 10a of the embodiment shown in FIG. 3. As for the structure that advances the driving unit and the blocker, a detailed description has been given with reference to FIG. 4 and FIGS. 5a to 5f, and therefore a detailed description thereof is omitted hereinafter. Meanwhile, in order to retract the piston 21, as shown in FIG. 1 or FIG. 16, an elastic member pressing the piston head 21a of the piston 21 toward the compressed gas tank 200 may be provided, and the piston 21 may be retracted using the pressing force of the elastic member. Alternatively, an exhaust passage for exhausting the compressed gas supplied by the compressed gas tank 200 may be provided, and a valve may be installed in the exhaust passage. After the piston 21 is advanced by the compressed gas, the piston 21 may be retracted using a pressure difference inside the housing 10a by exhausting the supplied compressed gas by opening the valve. As for this structure, reference is made to the embodiment to be described with reference to FIG. 11.

The embodiment shown in FIG. 11 is identical to the example shown in FIG. 4, except for the power generation structure of the driving unit 10. Therefore, the same reference numerals are assigned to structures identical to those in the embodiment shown in FIG. 4, and redundant descriptions are omitted.

Unlike the embodiment shown in FIG. 4, in the embodiment shown in FIG. 11, the driving unit-side plunger 12 is driven using compressed air delivered through a compressed air supply unit such as a compressed gas tank 17.

To this end, unlike the embodiment shown in FIG. 4, the internal chamber 10b of the housing 10a is in fluid communication with the compressed gas tank 17 through an inlet passage 102, instead of being wound with the solenoid coil 11 on its outer periphery. Further, the inlet passage 102 branches into a discharge passage 103 for discharging compressed air supplied from the compressed gas tank 17 to the internal chamber 10b to the outside of the needleless syringe. A valve 104 may be provided at a branching point between the inlet passage 102 and the discharge passage 103. The valve 104 controls opening and closing of an inflow channel through which compressed air is supplied to the internal chamber 10b through the inlet passage 102, and a discharge channel through which compressed air is discharged from the internal chamber 10b to the outside through the discharge passage 103. To this end, preferably, the valve 104 may be a, electronically controllable three-way valve.

The internal chamber 10b has at least one through-hole 10c positioned behind the point where the driving unit-side plunger 12 collides with the piston head 21a of the piston 21 when the driving unit-side plunger 12 advances due to the pressure by compressed air. The through-hole 10c may be provided to penetrate the inner and outer peripheries of the hollow cylindrical shape forming the internal chamber 10b. Through the through-hole 10c, the internal chamber 10b is in fluid communication with a compression chamber 105, which is a space between the outer periphery of the internal chamber 10b and the inner periphery of the housing 10a. The compression chamber 105 is a space sealed from the exterior of the housing 10a and is configured to be in fluid communication with the internal chamber 10b only through the through-hole 10c.

Further, at the rear end of the internal chamber 10b, a magnet 106 may be provided as a holding member for holding the plunger 12 at a specific position. Here, the magnet 106 may be a permanent magnet or an electromagnet. The magnet 106 can serve to hold the driving unit-side plunger 12 at the rear end of the internal chamber 10b using a magnetic force when the driving unit-side plunger 12, made of metal, retracts in the internal chamber 10b. Due to the presence of the magnet 106, it becomes possible to accurately maintain the retracted position of the driving unit-side plunger 12 when the driving unit-side plunger 12 retracts. The holding member is not limited to the magnet 106. For example, the holding member may be a unit that holds the plunger 12 at a specific position by physically engaging with the plunger 12.

Hereinafter, using the embodiment illustrated in FIG. 11, a mechanism that incrementally advances the piston 21 during reciprocation of the driving unit-side plunger 12 is described with reference to that shown in FIG. 11.

First, with the driving unit-side plunger 12 held at the rear end of the internal chamber 10b by the magnet 106, the valve 104 blocks the discharge channel toward the discharge passage 103 and opens the inflow channel from the compressed air tank 17 to the internal chamber 10b through the inlet passage 102. Accordingly, a compressed gas from the compressed air tank 17 is ejected behind the driving unit-side plunger 12 through the inlet passage 102. Due to the pressure of the compressed gas, the driving unit-side plunger 12 advances. Meanwhile, for smooth advancement of the driving unit-side plunger 12, the magnet 106 is made of an electromagnet such that a magnetic field of the magnet 106 disappears when compressed air is ejected.

Meanwhile, the air in the internal chamber 10b positioned in front of the driving unit-side plunger 12 is pushed forward as the driving unit-side plunger 12 advances, and the pushed-out air is discharged to the compression chamber 105 through the through-hole 10c. As described above, since the compression chamber 105 is a sealed space, the internal pressure of the compression chamber 105 becomes relatively high due to the air flowing inside through the through-hole 10c.

Further, as described above through the embodiment of FIG. 4, the driving unit-side plunger 12 that advances collides with the piston head 21a of the piston 21, and as a result, the piston 21 advances until it collides with the blocker 22a. Further, as the piston 21 advances, the piston end 21b of the piston 21 presses and moves the adjacent nozzle unit-side plunger 32a forward. The drug 100 in the chamber 31 of the nozzle 30 is compressed by the distance that the nozzle unit-side plunger 32a advances, and the drug is ejected in a predetermined amount through the nozzle hole 33 of the nozzle 30. Further, as described above with reference to FIG. 5d, the step motor 14 rotates the drive shaft 15, so the rotational force of the driving shaft 15 is transmitted to the blocker-side screw joint 24 through the power switching mechanism 24a, thereby advancing the blocker 22a by a predetermined distance.

Meanwhile, when the piston 21 collides with the blocker 22a, at that time point, the valve 104 closes the inflow channel through the inlet passage 102 and opens the discharge channel through the discharge passage 103. Accordingly, the compressed air behind the forward-positioned nozzle unit-side plunger 32a is discharged through the discharge passage 103. As a result, the pressure in the internal chamber 10b behind the nozzle unit-side plunger 32a decreases, thereby generating a pressure difference with the pressure inside the compression chamber 105. Due to this pressure difference, the compressed air in the compression chamber 105 flows through the through-hole 10c to the front of the nozzle unit-side plunger 32a, and the nozzle unit-side plunger 32a retracts due to the pressure of the inflowing compressed air. When the nozzle unit-side plunger 32a retracts near the position where the magnet 106 is installed, it is stopped at the position, where it comes into contact with the magnet 106, due to the magnetic field of the magnet 106. As described above, when the magnet 106 is configured as an electromagnet, the magnetic field of the magnet 106 is caused to be generated again at least at the time point when the nozzle unit-side plunger 32a collides with the piston 21 so that the nozzle unit-side plunger 32a, having collided with the piston 21, can be maintained at a predetermined retracted position by the magnet 106 when retracting retraction. Further, as described above with reference to FIG. 5f, the step motor 14 rotates the driving shaft 15, and the rotational force of the driving shaft 15 is transmitted to the housing-side screw joint 16a through the power switching mechanism 24a, thereby advancing the housing 10a by a predetermined distance. Therefore, when the valve 104 is subsequently opened, compressed air is supplied again from the compressed air tank 17, and the driving unit-side plunger 12 advances, the collision position between the driving unit-side plunger 12 and the piston 21 inside the housing 10a is maintained as described above, so even though the same amount of compressed air is supplied, the piston 21 can be advanced by a predetermined distance. Therefore, each time the driving unit-side plunger 12 reciprocates, a predetermined amount of the drug 100 can be ejected from the nozzle hole 33 of the nozzle unit 30.

Previously, a mechanism in which the reciprocating motion of the driving unit-side plunger 12 is converted into the incremental advancing motion of the piston 21 by advancing the housing 10a and the blocker 22a by rotating the step motor 14 was described, but embodiments of the present disclosure are not limited to the above configuration. That is, as described with reference to FIG. 3, instead of moving the housing 10a or the blocker 22a, it is also possible to incrementally advance the piston 21 by changing the amount of air supplied from the compressed air tank 17 each time the driving unit-side plunger 12 reciprocates. That is, for example, by adjusting the opening degree of the valve 104 so that more compressed air can be supplied to the internal chamber 10b of the housing 10a during the next reciprocating motion of the driving unit-side plunger 12 than during the previous reciprocating motion of the driving unit-side plunger 12, it is possible to apply a greater pressure to the driving unit-side plunger 12. Accordingly, the kinetic energy that is transmitted from the driving unit-side plunger 12 to the piston 21 each time the driving unit-side plunger 12 reciprocates can be kept identical. Therefore, in the same manner as when the housing 10a and the blocker 22a are moved, a predetermined amount of the drug 100 can be ejected from the nozzle hole 33 of the nozzle 30 each time the driving unit-side plunger 12 reciprocates.

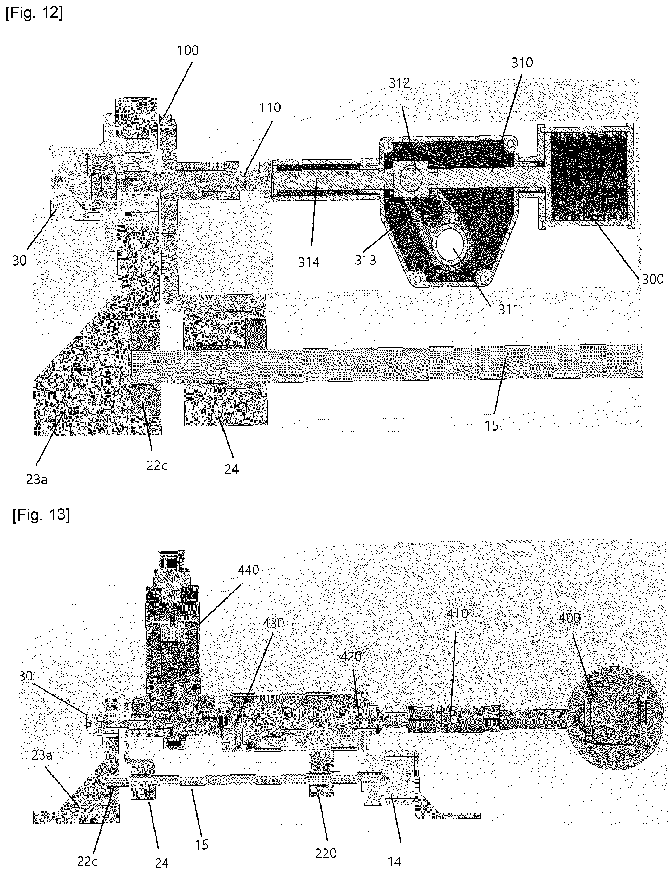

The embodiment shown in FIG. 12 is identical to the example shown in FIG. 4, except for the power generation structure of the driving unit 10. Therefore, the same reference numerals are assigned to structures identical to those in the embodiment shown in FIG. 4, and redundant descriptions are omitted.

In the example shown in FIG. 12, a spring, which is an elastic member, and a motor are used as a means for applying pressure to the piston 21.

In the embodiment shown in FIG. 12, a spring 300 is provided as an elastic member. A first end of the spring 300 is secured at a fixed position inside the needleless syringe main body, while a second end is provided at a first end of a first rod 310 extending in the longitudinal direction of the needleless syringe. A second rod 314 is provided at the front end that is a second end of the first rod 310, and the front end of the second rod 314 faces the piston head 21a of the piston 21. Here, the first rod 310 and the second rod 314 may be separate rods combined together or may be integrally formed. Meanwhile, any one of the first rod 310 or the second rod 320 is rotatably connected at the first end to a crank shaft 311 that is a rotary member, so it is rotatably connected to a second end of a crank arm 313 that rotates about the crank shaft 311 through a connecting portion 312. Further, the crank shaft 311 is configured to rotate by a predetermined angle by a rotary power source, such as a motor.

According to the above configuration, first, while the crank shaft 311 is rotated clockwise by a predetermined angle by a motor or the like, the rear end of the first rod 310 presses the spring 300. In this state, when the power source applied to the crank shaft 311 is cut off, the first rod 310 advances due to the elastic force of the spring 300, and accordingly, the front end of the second rod 320 strikes the piston 21. Further, at the time point when the piston 21 reaches the top dead center and collides with the blocker 22a, if the crank shaft 311 is rotated clockwise again by a motor or the like, the first rod 310 retracts again and its rear end presses the spring 300. In this state, as described above with reference to FIG. 4, by cutting off again the power source that is applied to the crank shaft 311 after moving the blocker 22a by a predetermined distance, the first rod 310 and the second rod 314 advance and collide with the piston head 21a, thereby advancing the piston 21. In the example of FIG. 4, the housing 10a is advanced by the same amount as the advancement of the blocker 22a; however, in the example shown in FIG. 10, by controlling the rotation amount of the crank shaft 311 by a motor or the like and adjusting the elastic force of the spring 300, the piston 21 can advance further by the same amount as the advancement of the blocker 22a. Alternatively, similar to the housing 10a shown in FIG. 4, it may be possible to advance the driving unit itself, which integrally has the spring 300, the motor, etc., using a screw joint.

Furthermore, similar to the mechanism described with reference to FIG. 3, by adjusting the elastic force applied by the spring 300 without advancing the blocker 22a or the housing 10a, it is possible to remain the kinetic energy that is transmitted from the front end of the second rod 320 to the piston 21 constant. That is, by controlling the elastic force that applied to the second rod 320 to increase in magnitude for each reciprocation of the second rod 320, it is possible, as in the case of moving the housing 10a and the blocker 22a, to eject a constant amount of the drug 100 from the nozzle hole 33 of the nozzle unit 30 each time the second rod 320 reciprocates. To this end, for example, by changing the compression amount of the spring 300 by adjusting the rotation amount of the crank shaft 311 rotating counterclockwise, it is possible to change the elastic force generated by the spring 300.

The embodiment shown in FIG. 13 is identical to the example shown in FIG. 3, except for the power generation structure of the driving unit 10. Therefore, the same reference numerals are assigned to structures identical to those in the embodiment shown in FIG. 3, and redundant descriptions are omitted.

In the example shown in FIG. 13, a compressed gas and a motor are used as a means for applying pressure to the piston 21.

In the example shown in FIG. 12, the crank arm 313 is driven using the spring 300 and unidirectional rotation of the motor; however, in the example shown in FIG. 13, the crank shaft, which is a rotating member provided inside a crank 410, is rotated solely by a motor using bidirectional rotation of the motor An end of a rod-shaped air compression piston 420 extending in the longitudinal direction of the needleless syringe extends into the crank 410, so it is rotatably connected to the crank arm that rotates with rotation of the crank shaft. Thus, as the crank shaft is rotated by the driving force of the motor 400, the air compression piston 420 can repeatedly reciprocate forward and backward. Further, a compressed air chamber 430 is provided in front of the air compression piston 420 and a valve 440 having the same structure as the valve 210 shown in FIG. 10 is provided in front of the compressed air chamber 430, thereby allowing or blocking the flow of compressed air from the compressed air chamber 430 toward the piston 21. The driving unit composed of the compressed air piston 420 and the motor 400, shown in FIG. 13, also, as in the example shown in FIG. 10, has a compressed gas tank-side screw joint 220 beneath it, so when the driving shaft 15 is rotated by the step motor 14, the driving unit and blocker can be advanced in the same manner as the housing 10a in the example shown in FIG. 4. The advancing and retracting of the piston 21 using compressed air was in detail with reference to FIGS. 10 and 11, and the advancing structure of the driving unit and the blocker was in detail with reference to FIG. 4; therefore, detailed description hereinafter is omitted.

Furthermore, as in the mechanism described with reference to FIG. 3, by adjusting the amount of compressed air from the compressed air chamber 430 toward the piston 21 without advancing the blocker 22a or the housing 10a, it is possible to eject a constant amount of the drug 100 from the nozzle hole 33 of the nozzle unit 30 each time the air compression piston 420 reciprocates, in the same manner as when the housing 10a and blocker 22a are moved. To this end, for example, by changing the amount of air, which is compressed by the air compression piston 420, by adjusting the rotation amount of the crank shaft 311, it is possible to change the elastic force generated by the spring 300.

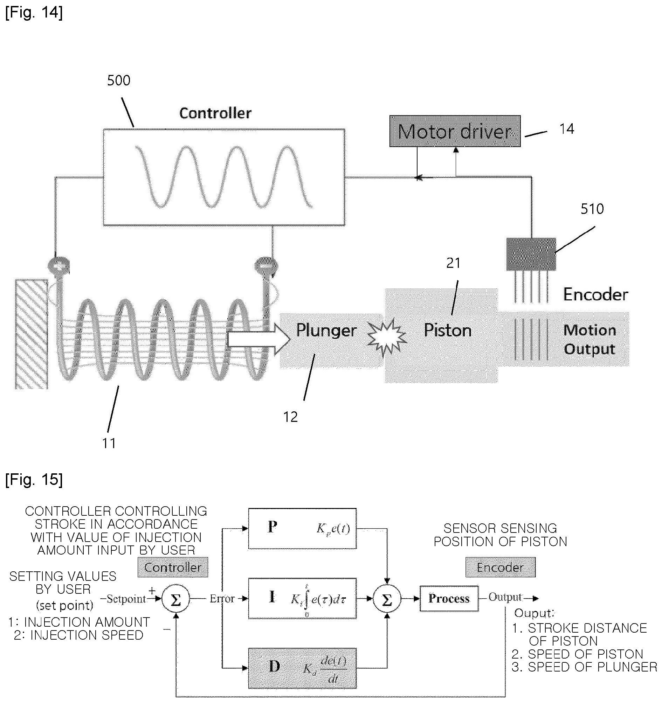

FIG. 14 is a diagram for explaining the position and speed control of a piston according to an exemplary embodiment of the present disclosure and FIG. 15 is a diagram for explaining PID control that is used for position and speed control of the piston shown in FIG. 14.

According to the embodiment shown in FIG. 14, for example, an encoder 510 capable of detecting the real-time position of the piston 21 is provided on the inner wall of the housing internal chamber 10b facing the piston 21, so a time-based position of the piston 21 is detected when the piston 21 advances and retracts. The result detected by the encoder 510 is transmitted to a controller 500. Meanwhile, in the controller 500, target injection amount and injection speed of a microjet-type drug to be discharged from the nozzle unit 30, as well as a stroke distance and a speed of the piston 21, a speed of the nozzle unit-side plunger, etc. necessary to satisfy the injection amount and injection speed, are pre-stored. Accordingly, the controller 500 performs PID control shown in FIG. 15 on the basis of: information regarding the target injection amount and injection speed of drug, and the stroke distance and speed of the piston 21 required to satisfy them; actual time-based position information of the piston 21 transmitted from the encoder 510; and the current position of the blocker 22a based on the number of rotations of the step motor 440. That is, after a single advancement of the piston, the stroke and speed of the piston during the single advancement of the piston are calculated on the basis of the information on the time-based position of the piston measured through the encoder 510 and the information on the current position of the blocker 22a measured through the rotation sensor of the step motor 440; errors between the calculated values and the target values of the stroke distance and speed of the piston 21 are calculated; and then the amount of current applied to the solenoid coil 11 and a control signal value applied to the step motor 440 to reduce the errors to zero are calculated. Further, a power source and a motor driving source are controlled so that the calculated amount of current and control signal are applied. Through feedback control that repeatedly performs this process, drug injection according to target injection amount and injection speed can be carried out.

Further, even in the case of a mechanism that controls the pressure that is applied to the nozzle unit-side plunger instead of the mechanism that moves the housing 10a and the blocker 22a using the step motor 14, the above PID control can be utilized. That is, after a single advancement of the piston, the changed piston position information is acquired through the encoder 510, and on the basis of the previously stored information regarding the target injection amount and injection speed of drug, as well as the stroke distance and speed of the piston 21 required to satisfy them, the current that is applied to the solenoid coil 11 during the next position advancement to satisfy the same stroke distance and speed as the previous advancement is calculated. Further, the power source can be controlled so that this current amount is satisfied during the next piston advancement.

The above controller may be implemented in the form of a computer provided in the needleless syringe. In that case, a program for implementing this control function may be recorded on a computer-readable recording medium, and the program recorded on the recording medium may be read into a computer system and executed to implement the function. Further, the "computer system" stated herein is a built-in computer system of a vehicle and may include OS or hardware of peripheral devices. Further, the term "computer-readable recording medium" refers to portable media such as flexible disks, optical magnetic disks, ROM, CD-ROM, and storage devices such as hard disks built in computer systems. Further, the "computer-readable recording medium" may also include an object that temporarily and dynamically hold programs such as communication lines when programs are transmitted through networks like the Internet or communication lines such as telephone lines, and an object that holds programs for a certain period such as volatile memory inside a computer system serving as a server or client. Further, the program may be for implementing a part of the functions described above, or may be implemented by combining the function described above with a program already recorded in a computer system.

### [Mode of Invention]

The present disclosure relates to a needleless syringe including a driving unit, a conversion unit, and a nozzle unit, in which the conversion unit causes a power transmission unit to reciprocate using pulse-shaped driving force generated by the driving unit, and converts a reciprocating motion of the power transmission unit into incremental advancing of a piston. Further, the incremental advancing motion of the piston causes the drug contained in a nozzle unit to be ejected from a nozzle hole of the nozzle unit in the form of a microjet.

Meanwhile, in a preferred embodiment of the present disclosure, each time the piston incrementally advances, the position of the power transmission unit is changed so that the relative position between the piston and the power transmission unit remains constant. Accordingly, it is possible to eject a predetermined amount of drug at a constant speed each time the power transmission unit reciprocates.

Further, in another exemplary embodiment of the present disclosure, by increasing the magnitude of a driving force each time the power transmission unit reciprocates, the kinetic energy transmitted from the power transmission unit to the piston is made constant each time the power transmission unit reciprocates. Accordingly, it is possible to eject a predetermined amount of drug at a constant speed each time the power transmission unit reciprocates.

### [Industrial Applicability]

According to the present disclosure, the drug in the nozzle can be ejected to the outside in a predetermined amount with an incremental advancing motion of the plunger, so the nozzle itself can be used as a cartridge without the need to provide a separate drug charger outside the nozzle and to periodically supply the drug from the drug charger into the nozzle. Therefore, the existing complex drug charging mechanism can be simplified. As a result, the manufacturing costs of a nozzle and a needleless syringe including the nozzle can be reduced. Further, according to the present disclosure, the drug in the nozzle can be incrementally ejected multiple times per second through the piston's incremental advancing motion repeated multiple times per second, whereby it is possible to reduce the problem that a large amount of drug remains inside the nozzle, unlike existing needleless syringes in which drug charging and ejection alternately occur through the reciprocating motion of a piston. Moreover, according to the present disclosure, when the piston completes advancing, a user can separate and discard a nozzle-integrated cartridge from the main body of the needleless syringe and can easily mount a new cartridge onto the main body. Therefore, even at home, a user can easily perform cartridge removal and replacement. Furthermore, according to the present disclosure, by changing the relative position of the blocker and the piston, the striking distance of the piston can be controlled, whereby it is possible to easily control the ejection speed and ejection amount of drug.

## Claims

1. A needleless syringe comprising a driving unit, a conversion unit, and a nozzle unit, wherein the driving unit provides a pulse pressure or a pulse-type driving force for reciprocating a power transmission unit accommodated in the housing;
the conversion unit converts the pulse pressure or a reciprocating motion of the power transmission unit into an incremental advancing motion of the piston such that, for each pulse of the pulse pressure or each reciprocating motion of the power transmission unit, the piston that has received kinetic energy from the driving unit incrementally advances by a predetermined distance toward the nozzle unit; and
the nozzle unit has a drug reservoir containing a drug therein, and discharges the drug contained in the drug reservoir in the form of a microjet through a nozzle hole in accordance with pressure applied to the drug by the piston.

2. The needleless syringe of claim 1, wherein the piston receives the kinetic energy by receiving the pulse-type pressure or by colliding with the power transmission unit; and
the conversion unit ensures that the kinetic energy transmitted to the piston is constant for each pulse or each reciprocating motion of the power transmission unit by advancing the driving unit by a predetermined distance each time the piston advances by the predetermined distance due to each pulse of the pulse pressure or one reciprocating motion of the power transmission unit.

3. The needleless syringe of claim 1, wherein the piston receives the kinetic energy by receiving the pulse-type pressure or by colliding with the power transmission unit; and
the conversion unit ensures that the kinetic energy transmitted to the piston is constant for each pulse or each reciprocating motion of the power transmission unit by increasing the magnitude of the pulse pressure or a driving force that is applied to the power transmission unit through the driving unit each time the piston advances by the predetermined distance due to each pulse of the pulse pressure or one reciprocating motion of the power transmission unit.

4. The needleless syringe of claim 2, wherein the power transmission unit is a plunger accommodated in the housing;
the housing has an internal chamber in a hollow internal space of the housing, and parts of the plunger and the piston are accommodated in the internal chamber;
a blocker is provided at a nozzle unit-side front end of the housing to limit an advancing motion of the piston that advances toward the nozzle unit by collision with the plunger; and
the needleless syringe further comprises a displacing unit capable of advancing the housing and the blocker at least toward the nozzle unit and a controller configured to control the displacing unit.

5. The needleless syringe of claim 3, wherein the power transmission unit is a plunger accommodated in the housing;
the housing has an internal chamber in a hollow internal space of the housing, and parts of the plunger and the piston are accommodated in the internal chamber;
a blocker is provided at a nozzle unit-side front end of the housing to limit an advancing motion of the piston that advances toward the nozzle unit by collision with the plunger; and
the needleless syringe further comprises a controller configured to control a driving force generated by the driving unit so that kinetic energy that is transmitted from the plunger to the piston is constant each time the plunger reciprocates.

6. The needleless syringe of claim 4 or 5, wherein the nozzle unit is configured to be detachable coupled to a main body of the needleless syringe comprising at least the housing and the blocker; and
the nozzle unit-side end of the piston extends into a chamber constituting the drug reservoir of the nozzle unit.

7. The needleless syringe of claim 6, wherein the chamber of the nozzle unit is configured such that one surface facing the main body of the needleless syringe is open to the outside; and
a first cover is detachably provided on the nozzle unit to be able to cover the open surface of the chamber when the nozzle unit is separated from the main body of the needleless syringe.

8. The needleless syringe of claim 7, wherein a second cover made of a material that can be cut by the end of the piston when the end advances is further provided on an inner side of the first cover facing the chamber; and
a nozzle unit-side plunger configured to pressurize a drug contained in the chamber is further provided inside the second cover.

9. The needleless syringe of claim 4 or 5, wherein the driving unit comprises a solenoid coil wound around an outer periphery of the internal chamber of the housing; and
the controller is configured to provide a pulse reciprocating motion of the plunger by controlling a current that is applied to the solenoid coil.

10. The needleless syringe of claim 1, further comprising:
a diaphragm configured to deform with the pulse pressure or a reciprocating motion of the power transmission unit;
a conversion unit-side chamber of which an internal pressure increases or decreases with deformation of the diaphragm;
a driving fluid supply unit being in fluid communication with the conversion unit-side chamber through a driving fluid supply passage and configured to supply a driving fluid to the conversion unit-side chamber as the internal pressure of the conversion unit-side chamber increases or decreases with deformation of the diaphragm; and
a check valve configured to allow only unidirectional flow of the driving fluid from the conversion unit-side chamber toward the internal chamber of the housing,
wherein the piston is configured to incrementally advance toward the nozzle unit by a hydraulic pressure of the driving fluid that is supplied from the driving fluid supply unit to the internal chamber of the housing.

11. The needleless syringe of claim 2 or 3, wherein the driving unit further comprises:
a compressed gas tank configured to supply a compressed gas into the internal chamber of the housing through an inflow passage; and
a valve provided on the inflow passage and configured to control inflow and outflow of the compressed gas into and from the internal chamber, and
the driving unit is controlled by the controller to provide the pulse pressure.

12. The needleless syringe of claim 4 or 5, wherein the driving unit further comprises:
a compressed gas tank configured to supply a compressed gas into the internal chamber of the housing through an inflow passage; and
a valve provided on the inflow passage and configured to control inflow and outflow of the compressed gas into and from the internal chamber, and
a discharge passage configured to discharge a compressed gas flowing inside through the inflow passage to the outside of the needleless syringe branches from the inflow passage and the valve adjusts opening and closing of the inflow passage and the discharge passage;
the internal chamber has at least one through-hole positioned behind a point at which the plunger first collides with a piston head of the piston when the plunger advances due to pressure of the compressed gas;
the internal chamber is in fluid communication with a compression chamber outside the internal chamber through the through-hole;
as the plunger advances, air discharged from the internal chamber through the through-hole is compressed in the compression chamber;
when the discharge passage is opened by the valve and the compressed gas is discharged to the outside of the needleless syringe after the plunger collides with the piston, the air compressed in the compression chamber flows back into the internal chamber through the through-hole due to a pressure difference between pressure of the internal chamber and pressure of the compression chamber; and
the plunger is moved to an original retracted position, from which the plunger started advancing, by the air flowing back into the internal chamber.

13. The needleless syringe of claim 12, further comprising a holding member configured to hold the plunger at the retracted position,
wherein the holding member holds the plunger at the retracted position until the plunger advances due to the pressure of the compressed air.

14. The needleless syringe of claim 2 or 3, comprising:
a rod having a first end facing a piston head of the piston;
an elastic member having a first end in contact with a second end of the rod and having a second end fixed to a main body of the needleless syringe; and
a rotating member configured to rotate by a predetermined angle by a driving force generated by the driving unit, and having an end rotatably connected to the rod,
wherein when the rotating member is rotated by the driving force in a direction in which the second end of the rod compresses the elastic member, and then the driving force is removed, the first end of the rod strikes the piston head while the rod advances by an elastic force of the elastic member, thereby incrementally advancing the piston.

15. The needleless syringe of claim 2 or 3, further comprising:
a compressed air chamber configured to supply compressed air into the internal chamber of the housing in the form of the pulse pressure;
an air-compressing piston configured to compress air in the compressed air chamber through forward and backward reciprocating motions;
a rotating member configured to rotate by a predetermined angle by a driving force generated by the driving unit, and having a first end rotatably connected to the air-compressing piston; and
a valve configured to control inflow and outflow of the compressed air into and from the internal chamber,
wherein, as the rotating member rotates, the air-compressing piston compresses air in the compressed air chamber while repeatedly reciprocating forward and backward, and flow of compressed air from the compressed air chamber toward the piston is controlled by opening and closing the valve, whereby the piston is incrementally advanced by pressure of the compressed air.

16. The needleless syringe of claim 4, further comprising an encoder capable of detecting a real-time position of the piston,
wherein information on a stroke distance and a speed of the piston required to satisfy target injection amount and injection speed of a drug that is discharged from the nozzle unit is stored in advance in the controller;
the controller calculates control signal values required to control the driving unit and the displacing unit to obtain the target injection amount and the injection speed of the drug, on the basis of real-time position information of the piston transmitted from the encoder and the information on the stroke distance and the speed of the piston stored in advance; and
the controller controls the driving unit and the displacing unit on the basis of the calculated control signal values.

17. The needleless syringe of claim 5, further comprising an encoder capable of detecting a real-time position of the piston,
wherein information on a stroke distance and a speed of the piston required to satisfy target injection amount and injection speed of a drug that is discharged from the nozzle unit is stored in advance in the controller;
the controller calculates a control signal value required to control the driving unit to obtain the target injection amount and injection speed of the drug, on the basis of real-time position information of the piston transmitted from the encoder and the information on the stroke distance and the speed of the piston stored in advance; and
the controller controls the driving unit on the basis of the calculated control signal value.
